Europäisches Patentamt

European Patent Office

Office européen des brevets

(11) Numéro de publication: **0 192 588**
**A1**

## DEMANDE DE BREVET EUROPEEN

(21) Numéro de dépôt: **86420033.2**

(22) Date de dépôt: **31.01.86**

(51) Int. Cl.⁴: **A 61 M 1/16**

(30) Priorité: **01.02.85 FR 8501604**

(43) Date de publication de la demande:
**27.08.86 Bulletin 86/35**

(84) Etats contractants désignés:
**BE CH DE GB IT LI NL SE**

(71) Demandeur: **CLINIQUE UNIVERSITAIRE GASTHUISBERG**
**Sce Médecine Interne Div. Néphrologie**
**Louvain(BE)**

(72) Inventeur: **Hauglustaine, Didier**
**2, Weligerveld**
**B-3041 Pellenberg (Louvain)(BE)**

(74) Mandataire: **Gauckler, Jacques et al,**
**Service Brevets HOSPAL HOSPAL C.O.T. B.P. 21**
**F-69881 Meyzieu Cedex(FR)**

(54) **Rein artificiel et produit à mettre en oeuvre dans un traitement extracorporel du sang.**

(57) Ce rein artificiel comprend des moyens (12) pour porter au contact du sang circulant dans le circuit extracorporel de sang (11) une première solution (25) contenant la totalité du bicarbonate de sodium, à titre d'agent tampon, et éventuellement des sels minéraux autres que ceux susceptibles de faire précipiter le bicarbonate, et des moyens (17, 19-23) pour porter séparément au contact du sang circulant dans le circuit extracorporel (11) une seconde solution de nature et de composition complémentaires.

EP 0 192 588 A1

"Rein artificiel et produit à mettre en oeuvre dans un traitement extracorporel du sang.".

La présente invention concerne un rein artificiel comprenant un circuit extracorporel de sang traversant un premier compartiment d'un dispositif d'hémodialyse, d'hémofiltration ou d'hémodiafiltration, séparé par une membrane semi-perméable d'un second compartiment. Elle concerne également un produit à mettre en oeuvre dans le traitement extracorporel du sang.

Les reins artificiels utilisés jusqu'à présent comportent des moyens permettant de traiter le sang soit par hémodialyse, soit par hémofiltration, soit encore par hémodiafiltration. Et dans ces différents cas, on utilise respectivement soit un liquide de dialyse , soit un liquide substitution de composition semblable à celle d'un liquide de dialyse, mais stérile et apyrogène, soit une combinaison de ces deux liquides.

Ces différents liquides contiennent généralement et pendant toute la durée du traitement tous les éléments nécessaires au maintien, ou le cas échéant au rétablissement, des équilibres électrolytiques et acido-basique du patient. En particulier, outre les sels minéraux tels que les sels de sodium, calcium, magnésium et potassium, nécessaires à l'équilibre électrolytique, ils contiennent toujours, au moins en partie soit un élément tampon tel que le bicarbonate, soit un élément précurseur d'élément tampon, tel que l'acétate, qui n'agit comme élément tampon qu'après sa transformation en bicarbonate, dans l'organisme du patient.

On a constaté que les traitements à l'acétate présentaient divers inconvénients. D'abord la transformation de l'acétate en bicarbonate n'est pas immédiate, d'où, chez le patient, au début de chaque traitement, un déficit initial d'éléments alcalins (acidose) et d'oxygène (hypoxie). En outre, entre deux traitements consécutifs, la décomposition de l'acétate en bicarbonate peut être irrégulière, d'où parfois une persistance du manque d'éléments alcalins (acidose). Il en résulte que le patient peut éprouver des troubles tels que nausées, maux de tête, instabilités de la tension, etc...

Si l'utilisation directe du bicarbonate permet d'éliminer la plupart de ces malaises, elle entraîne par contre, en raison des risques de précipitation du bicarbonate en présence de sels de calcium et de magnésium, des conditions de mise en oeuvre généralement plus délicates et plus coûteuses, nécessitant des systèmes de pompage plus complexes. En outre, dans certaines conditions, la teneur du liquide de dialyse en bicarbonate peut être irrégulière.

Aussi est-ce un objet de la présente invention que de proposer un rein artificiel et un produit qui ne présentent pas les inconvénients de l'art antérieur.

Plus particulièrement, un objet de la présente invention est, d'une part, d'éviter chez le patient les malaises susceptibles d'être engendrés par l'acétate et, d'autre part, d'éviter les inconvénients technologiques entraînés par la précipitation du bicarbonate.

C'est en effet un objet de la présente invention d'éliminer les conditions de la précipitation du bicarbonate et de proposer une fiabilité sensiblement améliorée, tant des solutés que de l'appareillage employés.

Un autre objet de la présente invention est de proposer un rein artificiel qui soit d'une mise en oeuvre simple, et plus économique avec notamment des volumes de concentrés réduits, d'un fonctionnement automatique et qui présente toutes les garanties de sécurité désirées.

Encore un autre objet de la présente invention est de proposer un rein artificiel d'une efficacité très nettement améliorée, c'est-à-dire qui permette une purification du sang , sans apparition de trouble chez le patient.

Un autre objet de l'invention consiste en la mise en oeuvre d'un circuit de liquide de dialyse qui n'est pas rendu compliqué, notamment par la quantité de liquide à extraire par ultrafiltration, en plus de celle normalement requise pour la diminution de poids du patient.

Pour résoudre ces problèmes, il est prévu, suivant l'invention, un produit approximativement isotonique du sang comprenant une première solution aqueuse contenant du bicarbonate de sodium, à titre d'agent tampon, ainsi qu'éventuellement des sels minéraux autres que ceux susceptibles de faire précipiter le bicarbonate de sodium, et une deuxième solution aqueuse de composition et nature complémentaires à la première solution, contenant notamment la totalité des sels de calcium et magnésium du produit, ainsi qu'éventuellement des sels minéraux, autres que du bicarbonate de sodium, comme produit de combinaison pour une utilisation séparée des deux solutions dans un traitement extracorporel du sang.

Suivant l'invention, il est également prévu un rein artificiel tel que décrit au début et caractérisé en ce qu' il comprend des moyens pour porter au contact du sang circulant dans le circuit extracorporel une première solution d' une nature et d'une composition déterminées, contenant la totalité du bicarbonate de sodium, à titre d'agent tampon, et au moins une partie des sels minéraux autres que ceux susceptibles de faire précipiter le bicarbonate, et constituant ainsi la première fraction d'un liquide à mettre en oeuvre pour un traitement extracorporel du sang, et des moyens pour porter séparément au contact du sang circulant dans ledit circuit extracorporel une seconde solution, de nature et de composition complémentaires à celles de ladite première solution et constituant ainsi la seconde fraction du liquide de traitement extracorporel du sang, ladite seconde solution contenant notamment la totalité des sels de calcium et de magnésium, et éventuellement des sels minéraux autres que le bicarbonate de sodium.

La présente invention concerne donc un rein artificiel comportant des moyens pour que ladite première solution, circulant dans le second compartiment de l'hémodialyseur, soit en réalité, et contrairement à tout liquide de dialyse connu jusqu'à présent, dépourvue de tout ion magnésium et calcium, ainsi que de tout élément précurseur d'élément tampon, tel que des ions acétate.

Jusqu'à présent, on a utilisé, soit comme liquide de dialyse, soit comme liquide de substitution, des liquides isotoniques au sang à la fois sur le plan électrolytique et sur le plan de l'équilibre acido-basique pour des raisons évidentes de sécurité. Car tout médecin sait qu'un patient pourrait ne pas supporter un seul traitement de dialyse dans lequel l'équilibre acidobasique ne serait pas assuré. Il sait également qu'une absence totale d'ions calcium n'est pas envisageable, car elle conduirait à une décalcification du patient.Et le problème de la précipitation du bicarbonate en présence de calcium n'a jamais été résolu de manière satisfaisante.

Un rein artificiel comporte des moyens pour exercer essentiellement, d'une part, les fonctions d'épuration et de rétablissement et/ou de maintien de l'équilibre électrolytique et, d'autre part, la fonction de rétablissement et/ou de maintien de l'équilibre acido-basique du patient. Le rein artificiel suivant l'invention comporte des moyens distincts permettant de faire agir sur le sang séparément, en deux zones différentes, aussi proches soient-elles l'une de l'autre, deux solutions constituant deux fractions complémentaires d'un liquide à mettre en oeuvre dans le traitement du sang. Les deux solutions, bien qu'appliquées séparément, présentent ensemble une finalité de thérapie qui établit une fusion fonctionnelle des deux constituants, dans l'application visée. Indépendamment l'une de l'autre, elles ne peuvent produire l'effet avantageux conforme à l'invention ; elles ne le peuvent d'ailleurs pas plus si elles sont utilisées simultanément en mélange intime, car, dès ce moment, les inconvénients de la technique antérieure resurgissent et, en particulier, ceux résultant de la précipitation du bicarbonate de sodium.

Le maintien de l'équilibre acido-basique est essentiellement assuré par hémodialyse, hémofiltration ou hémodiafiltration, à l'aide de la première solution. Avantageusement cette première solution comprend, outre des ions bicarbonate, des sels minéraux nécessaires à l'équilibre électrolytique du patient, autres que ceux de calcium et de magnésium.

La seconde solution comprend notamment la totalité des ions calcium et magnésium, ainsi qu'éventuellement en plus des sels minéraux nécessaires à l'équilibre électrolytique du patient. Elle ne contient pas de bicarbonate de sodium.

Ainsi le rein artificiel selon la présente invention comporte-t-il des moyens distincts pour faire agir sur le sang séparément, d'une part, l'ensemble des sels de calcium et de magnésium contenus dans la deuxième solution et, d'autre part, l'ensemble des ions bicarbonate constituant l'élément tampon contenu dans la première solution. De cette manière, on évite tout risque de précipitation du bicarbonate.

Le rein artificiel selon la présente invention est pourvu de moyens permettant notamment de neraccorder le circuit du liquide de traitement et le circuit extracorporel de sang respectivement qu'à une source de liquide de traitement d'une composition spécialement prévue à cet effet et à un circuit comprenant une réserve de liquide de perfusion de composition complémentaire.

Dans le rein artificiel suivant l'invention, il est avantageusement prévu que lesdits moyens pour porter la seconde solution au contact du sang circulant dans le circuit extracorporel sont agencés pour perfuser directement dans le circuit extracorporel de sang environ 30 cm3/h de ladite seconde solution.

Suivant une forme avantageuse de réalisation de l'invention, les moyens pour faire circuler ladite première solution dans ledit second compartiment comprennent uniquement un moyen constituant une source de première solution, un moyen de circuit de la première solution raccordant ledit moyen de source au second compartiment, un moyen de circuit de la première solution raccordant le second compartiment à l'égoût, un moyen de pompage pour mettre en circulation la première solution dans son circuit et un moyen de pompage occlusif pour extraire de ce circuit un montant de liquide qui est égal ou supérieur au montant introduit dans ce circuit et qui est déterminé indépendamment du débit de seconde solution perfusé directement dans le sang.

Etant donné le faible débit de 30 cm3/h de la solution de sels de magnésium et de calcium perfusée directement dans le circuit extracorporel de sang, on peut se permettre de ne pas tenir compte de cette quantité négligeable de liquide introduite. Par conséquent, s'il est important qu'il subsiste un asservissement des moyens de pompage destinés à l'introduction de la seconde solution dans le circuit extracorporel aux moyens pour faire circuler le sang dans le circuit extracorporel, un asservissement des moyens de pompage occlusifs destinés à l'évacuation de la première solution du circuit de cette dernière aux moyens de pompage susdits, destinés à l'introduction de la seconde solution dans le circuit extracorporel, n'est pas nécessaire. Il en résulte une grande simplification d'appareillage et de processus de traitement.

Le rein artificiel selon la présente invention comporte encore avantageusement des moyens d'alarme et/ou de sécurité de tous types connus en soi, permettant de s'assurer par exemple que les diverses pompes fonctionnent normalement, aux vitesses prévues, que les débits des différents liquides restent dans les limites prévues, que les pressions restent dans les points les plus importants du circuit également dans des intervalles prédéterminés et que l'osmolarité ou les caractéristiques physiques, telles que le pH, des différents liquides restent comprises dans des intervalles prédéterminés.

Naturellement, le technicien peut ajouter tout dispositif de sécurité de type connu en soi, susceptible par exemple d'arrêter le traitement et d'isoler momentanément le circuit à l'aide de pinces et/ou d'un by-pass, pour le cas où une défaillance quelconque serait détectée.

Une telle défaillance pourrait être provoquée notamment par une chute brutale de la pression artérielle, c'est-à-dire à l'entrée du circuit extracorporel de sang, observée par exemple en cas de rupture accidentelle du circuit, ou bien par une pression veineuse excessive à la sortie du circuit, par exemple due à un bouchage ou au pincement inopportun d'un tube du circuit de sang.
Elle peut être aussi provoquée par une grave irrégularité de l'écoulement qualitatif et/ou quantitatif des deux solutions . Ces défaillances peuvent être détectées par tous moyens connus et transmises aussitôt à un programmateur, qui peut alors agir automatiquement sur les pinces et/ou sur le by-pass, ainsi que sur la commande d'arrêt des diverses pompes.

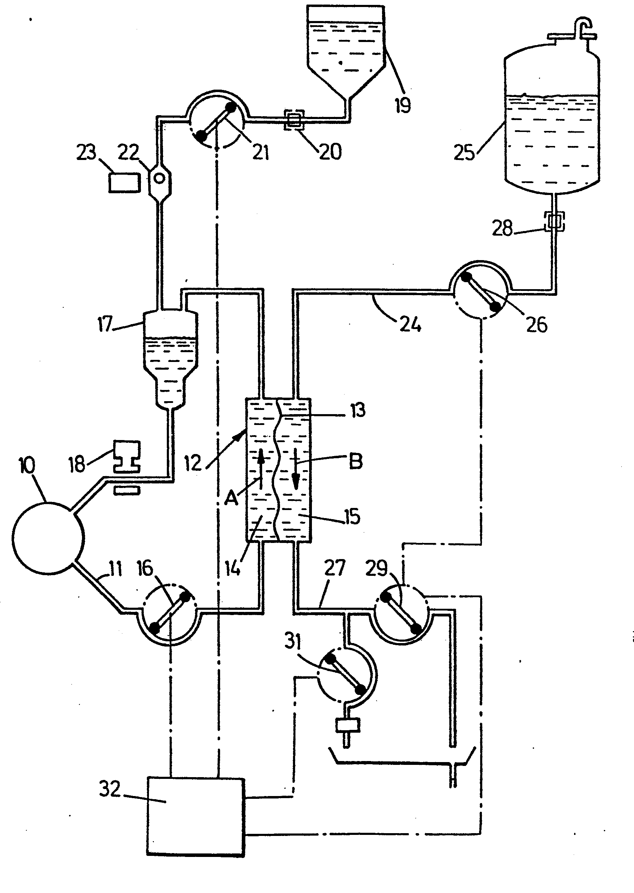

La compréhension de la présente invention sera facilitée par la figure unique ci-jointe qui illustre à titre d'exemple, schématiquement et sans échelle déterminée, une forme de réalisation préférée de rein artificiel selon la présente invention.

On peut voir sur la figure que le patient (10) est relié par un circuit extracorporel de sang (11) à un dispositif de traitement du sang (12) de tout type connu en soi ; dans ce cas particulier, il s'agit d'un hémodialyseur, mais il pourrait également s'agir entre autres d'un dispositif d'hémofiltration ou d'hémodiafiltration. Cet hémodialyseur est divisé par une membrane (13), semi-perméable et permettant des échanges notamment par dialyse

et par ultrafiltration, en deux compartiments (14) et
(15). Le compartiment (14) est parcouru par le sang et
le compartiment (15) est parcouru, de préférence à contre-
courant, par une première solution. Une pompe (16), par
exemple de type péristaltique, assure la circulation du
sang dans le sens indiqué par la flèche A. Sur la ligne
de retour du sang au patient, en aval de l'hémodialyseur
(12), un dispositif débulleur (17) de type connu en soi
et muni généralement d'un filtre (non représenté) retient
toute bulle d'air ou toute impureté. Enfin, en aval du
débulleur (17), une pince (18) ou tout dispositif d'obturation de type connu en soi est disposé juste avant le
retour du sang au patient (10).

Un réservoir (19), contenant une deuxième solution, maintenue stérile, et constitué par exemple par un
sac plastique souple de type connu en soi, est relié par
un connecteur (20) d'une type spécial, c'est-à-dire non
susceptible d'être relié à un embout d'un type standard,
(par exemple de formes connues, mais de dimensions hors
normes), à une pompe de perfusion occlusive (21) par exemple de type péristaltique, à vitesse réglable. Celle-ci
est reliée à un dispositif de compte-gouttes (22) de type
connu en soi, relié à un dispositif de comptage totalisateur (23). Le compte-gouttes (22) est à son tour relié au
circuit extracorporel de sang (11), préférentiellement à
l'aval de l'hémodialyseur (12), pour éviter de dialyser
immédiatement la solution perfusée. Avantageusement, il
est relié au débulleur (17).

Préférentiellement, le réservoir (19) et les conduits le
reliant au débulleur (17) sont d'un type prévu pour un
usage unique, et ils peuvent être fournis en association
avec les lignes constituant notamment le circuit extracorporel de sang.

Le compartiment (15) de l'hémodialyseur (12) est relié à un réservoir (25) par un conduit (24) d'un circuit parcouru par une première solution déplacée dans le sens de la flèche B par une pompe de circulation (26), par exemple de type péristaltique. Le réservoir (25) a une capacité suffisante pour permettre le traitement complet d'un patient sans renouvellement de ladite solution. Celle-ci est introduite avant le traitement dans le réservoir (25) par un conduit muni d'un embout (non représenté). Le réservoir (25) est raccordé au conduit (24), qui est muni d'un embout spécial (28), susceptible d'être relié exclusivement à un connecteur solidaire du réservoir contenant la première solution. Cet embout (28) est, de préférence, d'un type de forme connue, mais, grâce à des dimensions hors normes, il ne peut être relié ni au connecteur (20), ni à aucun autre connecteur du commerce. Cette incompatibilité est créée volontairement de manière à éviter tout risque de confusion possible quant à la nature des deux solutions différentes mises en oeuvre.

Une pompe occlusive (29), par exemple également de type péristaltique, est raccordée au deuxième compartiment (15) par un conduit (27) et elle permet d'extraire hors du circuit de la première solution un montant de liquide égal au montant de liquide introduit par la pompe (26) au cours du traitement. Les deux pompes (26) et (29) peuvent pour ce faire être reliées en série à un programmateur (32) qui asservit leur fonctionnement.

Une pompe occlusive (31), par exemple également de type péristaltique, peut être éventuellement prévue pour extraire hors du circuit (24), donc indirectement hors du circuit extracorporel de sang (11), par ultrafiltration à travers la membrane (13), un montant (volume et/ou débit) de liquide déterminé selon les conditions du traitement. Il est évident qu'on pourrait aussi prévoir de supprimer cette deuxième pompe (31) et de régler à une vitesse un peu plus élevée la pompe (29) pour obtenir le même résultat.

On règle avantageusement le débit de la pompe de perfusion (21) sur celui du débit du sang fourni par la pompe de circulation de sang (16). On maintient généralement constant le rapport du débit de la solution perfusée au débit de sang. Le programmateur (32), de type connu en soi, assure l'asservissement des débits moyens respectifs des pompes (16) et (21).

Pour faciliter la compréhension de la présente invention, on n'a décrit ici que les éléments essentiels du rein artificiel jouant un rôle selon l'invention. C'est ainsi que l'on ne fait pas mention des organes de chauffage du dialysat, car ils ne sont pas critiques en rapport avec la présente invention.

La première solution, qui traverse l'hémodialyseur (12) est essentiellement constituée d'un élément tampon, du bicarbonate, et elle est essentiellement dépourvue de tout élément pouvant risquer de faire précipiter le bicarbonate, c'est-à-dire qu'elle est dépourvue d'ions calcium et magnésium. Elle est également dépourvue de tout élément précurseur ou générateur d'élément tampon, tel que l'acétate ; et elle a pour fonction d'assurer essentiellement l'équilibre acido-basique du sang et l'épuration du sang du patient par un processus de diffusion. Elle peut éventuellement contenir en supplément des ions minéraux qui ne font pas précipiter le bicarbonate, par exemple les ions $Na^+$ et $K^+$.

Une solution concentrée, qui convient bien pour constituer par dilution ladite première solution, peut avoir la composition suivante, donnée uniquement à titre d'exemple :

| | |
|---|---|
| $NaHCO_3$ | 25,280 g. |
| NaCl | 71,543 g. |
| KCl | 1,344 g. |
| $H_2O$ | en quantité suffisante pour former 1 litre. |

Après dilution du concentré dans de l'eau pour obtenir 12 l. de solution, on obtient les concentrations suivantes :

| | | |
|---|---|---|
| $Na^+$ | 137 | milliéquivalents/l |
| $K^+$ | 1,5 | " |
| $HCO_3^-$ | 35 | " |
| $Cl^-$ | 103,5 | " |

Le débit de la première solution dans le dispositif d'hémodialyse peut par exemple être de 500 ml/min.

La deuxième solution contient au moins la totalité des ions calcium et magnésium, nécessaires pour l'équilibre électrolytique du sang du patient. On peut, si désiré, y ajouter quelques additifs, tels que des sels de potassium et/ou de sodium, à l'exclusion toutefois de tout bicarbonate, ainsi que de tout acétate. Une telle solution peut être fournie sous emballage plastique à usage unique et elle peut se conserver aisément. A titre d'exemple, on peut donner, pour la deuxième solution, la composition suivante :

| | |
|---|---|
| $CaCl_2.2H_2O$ | 51,35 g |
| $MgCl_2.6H_2O$ | 21,18 g |
| $H_2O$ | en quantité suffisante pour faire un litre. |

Il est évident que les compositions données ci-avant peuvent subir de larges variations en fonction des besoins du patient.

Le fonctionnement du rein artificiel selon la figure est le suivant, après raccordement et remplissage préalable des différents circuits, y compris le circuit d'héparinisation du sang (non représenté). Le médecin prescrit le débit moyen du sang, par exemple 300 ml/minute, ce qui fixe la vitesse de rotation de la pompe (16), puis il affiche la perte de poids que doit subir le patient lors du traitement, ce qui permet de fixer la vitesse de la pompe (31). Le programmateur (32) asservit aussi les vitesses égales des pompes (26) et (29) à une vitesse choisie. Le programmateur asservit enfin la vitesse de la pompe (21) en fonction du débit obtenu par la pompe (16), de façon qu'un débit d'environ 30 cm3/h de la deuxième solution soient introduits uniformément au cours du traitement dans le circuit (11).

On constate ainsi que le rein artificiel selon la figure comporte des moyens pour porter séparément au contact du sang une première et une seconde solutions, qui ensemble constitueraient un liquide de dialyse de type connu, mais seraient incapables d'offrir les avantages obtenus par le produit appliqué séparément en deux solutions suivant l'invention.

Naturellement, d'autres variantes de réalisation à la portée du technicien peuvent être mises en oeuvre, sans pour autant sortir du cadre de la présente invention.

C'est ainsi que l'on pourrait amener le sang en contact avec lesdites première et deuxième solutions dans un dialyseur unique comprenant trois chambres séparées, l'une centrale pour le sang, en contact par une membrane semi-perméable avec chacune des deux autres chambres.

Dans l'une de ces dernières circulerait la première solution et, dans l'autre, la deuxième solution. On peut aussi envisager deux hémodialyseurs indépendants disposés en série. En outre, la présente invention peut être mise en oeuvre en utilisant la technique connue sous le nom de dialyse à aiguille unique, le sang s'écoulant à travers l'aiguille unique alternativement du patient vers l'hémodialyseur et en sens inverse.

Si l'on envisage un dispositif d'hémofiltration, lesdites solutions, stériles et apyrogènes, peuvent être amenées séparément au contact du sang par perfusion directe dans le circuit extracorporel de sang. Dans ce cas, le second compartiment du dispositif d'hémofiltration sert uniquement comme c'est l'habitude à l'ultrafiltration du sang.

Il est également devenu possible suivant l'invention de conserver l'appareillage en bon état de service pendant davantage de temps, étant donné l'absence quasi totale de précipitation du bicarbonate. L'usage d'acétate peut, en outre, être évité et les inconvénients associés à cet usage sont supprimés.

## REVENDICATIONS

1. Produit approximativement isotonique au sang comprenant une première solution aqueuse contenant du bicarbonate de sodium, à titre d'agent tampon, ainsi qu'éventuellement des sels minéraux autres que ceux susceptibles de faire précipiter le bicarbonate de sodium, et une deuxième solution aqueuse d'une composition et nature complémentaires à la première solution, contenant notamment la totalité des sels de calcium et de magnésium du produit, ainsi qu'éventuellement des sels minéraux autres que du bicarbonate de sodium, comme produit de combinaison pour une utilisation séparée des deux solutions dans un traitement extracorporel du sang.

2. Produit suivant la revendication 1, caractérisé en ce que la première solution contient, en plus des ions bicarbonate, des ions sodium, potassium et chlorure et en ce que la deuxième solution contient du chlorure de calcium et du chlorure de magnésium.

3. Produit suivant la revendication 2, caractérisé en ce que la première solution est un concentré à diluer dans l'eau dans un rapport de 1/12, dont la composition est la suivante :

| | |
|---|---|
| $NaHCO_3$ | env. 35 g |
| NaCl | env. 71 g |
| KCl | env. 1 g |
| $H_2O$ | en quantité suffisante pour faire 1 litre |

et en ce que la deuxième solution comprend au moins 120 cm3 d'un liquide dont la composition est la suivante :

$CaCl_2 . 2H_2O$    env. 51 g

$MgCl_2 . 6H_2O$    env. 21 g

$H_2O$    en quantité suffisante

pour faire 1 litre

4. Rein artificiel comprenant un circuit extracorporel de sang (11) traversant un premier compartiment (14) d'un dispositif d'hémodialyse, d'hémofiltration ou d'hémodiafiltration (12), séparé par une membrane semi-perméable (13) d'un second compartiment (15), caractérisé en ce qu'il comprend des moyens (24-27) pour porter au contact du sang circulant dans le circuit extracorporel une première solution d'une nature et d'une composition déterminées, contenant la totalité du bicarbonate de sodium, à titre d'agent tampon, et au moins une partie des sels minéraux autres que ceux susceptibles de faire précipiter le bicarbonate, et constituant ainsi la première fraction d'un liquide à mettre en oeuvre pour un traitement extracorporel du sang, et des moyens (17,19-23) pour porter séparément au contact du sang circulant dans ledit circuit extracorporel (11) une seconde solution, de nature et de composition complémentaires à celles de ladite première solution et constituant ainsi la seconde fraction dudit liquide de traitement extracorporel du sang, ladite seconde solution contenant notamment la totalité des sels de calcium et de magnésium, et éventuellement des sels minéraux autres que le bicarbonate de sodium.

5. Rein artificiel selon la revendication 4, caractérisé en ce qu'il comporte, à titre de moyens pour porter au contact du sang ladite première solution, des moyens (24-27) pour faire circuler cette première solution dans ledit second compartiment (15).

6. Rein artificiel selon la revendication 4, caractérisé en ce qu'il comporte, à titre de moyens pour porter au contact du sang ladite première solution, stérile et apyrogène, des moyens pour perfuser directement cette première solution dans le circuit extracorporel du sang (11).

7. Rein artificiel selon l'une des revendications 4 à 6, caractérisé en ce qu'il comporte, à titre de moyens pour porter au contact du sang ladite seconde solution, stérile et apyrogène, des moyens (17,19-23) pour perfuser directement cette seconde solution dans le circuit extracorporel de sang (11).

8. Rein artificiel selon l'une ou l'autre des revendications 4 et 5, caractérisé en ce qu'il comporte, à titre de moyens pour porter au contact du sang ladite seconde solution, des moyens de dialyse et/ou d'ultrafiltration à travers une membrane semi-perméable.

9. Rein artificiel selon la revendication 7, caractérisé en ce que lesdits moyens (17, 19-23) pour porter ladite seconde solution au contact du sang circulant dans ledit circuit extracorporel (11) sont agencés pour perfuser directement dans le circuit extracorporel de sang environ 30 cm3/h de ladite seconde solution.

10. Rein artificiel selon la revendication 5, caractérisé en ce que les moyens pour faire circuler ladite première solution dans ledit second compartiment (15) comprennent uniquement un moyen constituant une source de première solution (25), un moyen de circuit de la première solution (24) raccordant ledit moyen de source (25) au second compartiment (15), un moyen de circuit de la première solution (27) raccordant le second compartiment (15) à l'égoût, un moyen de pompage (26) pour mettre en circulation la première solution dans son circuit et un moyen de pompage occlusif (29, 31) pour extraire de ce circuit un montant de liquide qui est égal ou supérieur au montant introduit dans ce circuit et qui est déterminé indépendamment du débit de seconde solution introduit dans le sang.

11. Rein artificiel selon l'une quelconque des revendications 4 à 10, caractérisé en ce qu'il comporte des moyens (32) pour asservir les débits de ladite seconde solution introduite dans le sang du patient au débit de sang dans le circuit extracorporel de sang (11).

12. Rein artificiel selon l'une quelconque des revendications 4 à 11, caractérisé en ce qu'il comporte des moyens (20-23,28) permettant de ne raccorder le circuit extracorporel de sang (11) qu'à un moyen (19) constituant une source de ladite seconde solution et de ne raccorder le circuit de la première solution (24,27) qu'à un moyen (25) constituant respectivement une source de cette première solution.

13. Rein artificiel selon l'une quelconque des revendications 4 à 12, caractérisé en ce que lesdits moyens pour porter au contact du sang ladite seconde solution comprennent des moyens (19) de stockage de ladite solution dans des conditions stérile et apyrogène, une pompe de perfusion occlusive (21), des conduits de liaison et des moyens de raccordement de type spécial audit circuit extracorporel de sang, ainsi que des moyens de contrôle (22, 23) de l'écoulement effectif de ladite seconde solution situés sur lesdits conduits de liaison entre ladite pompe et ledit circuit extracorporel.

14. Rein artificiel selon l'une quelconque des revendications 4 à 13, caractérisé en ce que ledit circuit extracorporel de sang (11) est du type à aiguille unique.

**0192588**

Office européen des brevets

## RAPPORT DE RECHERCHE EUROPEENNE

Numéro de la demande

EP 86 42 0033

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | FR-A-2 431 864 (CORDIS COW CORP.) * Page 9, lignes 28-35 * | 1,2 | A 61 M 1/16 |
| Y | | 4,5,8 | |
| X | US-A-3 482 575 (C. CLAFF CO.) * Figure 2; colonne 5, lignes 1-6, 27-46 * | 4,5,7 | |
| A | DORLAND'S ILLUSTRATED MEDICAL DICTIONARY, édition 24, page 1404, W.B. Saunders Company, Philadelphia et Londres * Page 1404: "Ringer's solution" * | 4,5,7 | |
| X | EP-A-0 086 553 (P. VELTMAN) * En entier * | 1,2 | **DOMAINES TECHNIQUES RECHERCHES (Int. Cl.4)** A 61 M |
| Y | DE-A-2 851 929 (P. WEISS) * Figure 1; page 5, lignes 2,3 * | 4,5,8 | |
| A | US-A-3 962 075 (S. FIALKOFF et al.) * Résumé * | 4,5,8 | |

Le présent rapport de recherche a été établi pour toutes les revendications

| Lieu de la recherche | Date d'achèvement de la recherche | Examinateur |
|---|---|---|
| LA HAYE | 23-04-1986 | VEREECKE A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82